# FASCICULE DE BREVET EUROPEEN

(11) **EP 3 155 924 B1**
(45) Date de publication et mention de la délivrance du brevet: **10.07.2019**
(21) Numéro de dépôt: 16187718.8
(22) Date de dépôt: 07.09.2016
(51) Int. Cl.: A45D 34/02

(54) **FLACON ET PROCEDE DE FABRICATION D'UN TUBE PLONGEUR POUR FLACON**
FLACON UND HERSTELLUNGSVERFAHREN EINES TAUCHROHRS FÜR DEN FLACON
VIAL AND METHOD FOR MANUFACTURING A VIAL DIP TUBE

(30) Priorité: 15.10.2015 FR 1559829
(43) Date de publication de la demande: 19.04.2017
(73) Titulaire: Albéa le Tréport, 76470 Le Tréport (FR)
(72) Inventeur: ELMEGUENNI, Mohamed, 80130 FRIVILLE ESCARBOTIN (FR)
(74) Mandataire: Gevers & Orès

(56) Documents cités:
- FR-A1- 2 837 799
- US-A1- 2003 075 264
- US-A1- 2007 125 804
- US-A1- 2014 197 248

## Description

L'invention concerne le domaine des flacons contenant une solution, notamment des flacons comprenant un corps transparent ou au moins translucide et un tube plongeur immergé dans la solution pour permettre l'approvisionnement en solution d'un organe de distribution, par exemple une pompe.

Le tube plongeur est classiquement réalisé en un matériau polymérique qui est incolore, transparent et facilement extrudable sous la forme de tubes de petite dimension. Par ailleurs, les matériaux utilisés pour ledit tube doivent présenter des caractéristiques mécaniques adaptées pour permettre leur association au corps de pompe à des cadences importantes, et présenter une résilience suffisante pour retrouver une configuration sensiblement droite après enroulement autour d'une bobine de stockage. Par exemple, les matériaux utilisés selon l'art antérieur sont en polyoléfine, de type polypropylène ou polyméthylepentène (TPX).

Pour des raisons esthétiques, les parfumeurs souhaitent l'invisibilité du tube plongeur qui sinon balafrerait les flacons.

Pour rendre ces tubes invisible dans la solution, pour les produits parfumants il est connu de les réaliser en un matériau à base de polymère fluoré dont l'indice de réfraction est proche de celui de la solution. Il est également nécessaire que leur procédé de transformation assure une cristallinité inférieure à 13%. Ces polymères fluorés sont non seulement coûteux et peu disponible (un seul fournisseur au monde de ces matières) mais présentent également un risque pour la santé. Le fluor migre dans le liquide et devient inhalable lorsqu'il est pulvérisé par un système de distribution, donc mis en contact avec les muqueuses du système respiratoire. Les tubes en polymères fluorés sont difficiles à manipuler en raison de leur effet électrostatiques attirant les poussières, les autres particules et leur film d'emballage. Ces tubes ont également des performances mécaniques inférieures à celles des tubes en polyoléfines sans fluor. Ils mémorisent les courbures ou pliages lors des procédés d'assemblages ou des transports.

Il existe donc un besoin d'un flacon comprenant un tube plongeur ayant des caractéristiques qui contribuent à rendre le tube plongeur invisible dans une solution et ne présentant pas les inconvénients mentionnés ci-dessus.

A cet effet, la présente invention propose un flacon contenant une solution, ledit flacon comprenant un corps transparent ou au moins translucide qui est surmonté par une bague pourvue d'une ouverture sur laquelle un organe de distribution de la solution est associé, ledit organe de distribution comprenant un corps d'organe de distribution et un tube plongeur communiquant l'un avec l'autre, ledit tube étant immergé dans la solution pour permettre l'approvisionnement dudit organe de distribution en solution à distribuer, ledit tube plongeur comprenant un silicone de manière à ce que la différence entre l'indice de réfraction dudit tube et l'indice de réfraction de ladite solution soit inférieure ou égale à 0.04.

La présente invention propose également un flacon configuré pour contenir une solution, ledit flacon comprenant un corps transparent ou au moins translucide qui est surmonté par une bague pourvue d'une ouverture sur laquelle un organe de distribution de la solution est associé, ledit organe de distribution comprenant un corps d'organe de distribution et un tube plongeur communiquant l'un avec l'autre, ledit tube étant configuré pour être immergé dans la solution pour permettre l'approvisionnement dudit organe de distribution en solution à distribuer, ledit tube plongeur comprenant un silicone de manière à ce que l'indice de réfraction dudit tube soit compris entre 1,36 et 1,44.

L'utilisation d'un matériau comprenant un silicone dont l'indice de réfraction est proche de celui de la solution contenue dans le flacon permet d'obtenir un tube plongeur sensiblement invisible dans sa partie immergée dans la solution. En particulier, les solutions parfumantes telles les eaux de toilettes ont un indice de réfraction compris entre 1,37 et 1,39. En outre, ce matériau présente l'avantage de pouvoir être fabriqué facilement, en particulier par extrusion, en des tubes de petite dimension, et de présenter une résilience suffisante pour ne pas garder la mémoire de la courbure imposée lors du stockage en bobine. Ces caractéristiques présentent un intérêt industriel important puisque, le tube plongeur est introduit à haute cadence dans le flacon au travers de l'ouverture dont la dimension réduite nécessite une bonne fiabilité dans le positionnement dudit tube relativement à ladite ouverture. Or, la présence d'une courbure trop importante sur le tube ne permet de garantir ce positionnement, ni d'ailleurs de garantir le positionnement de l'extrémité inférieure du tube dans le flacon comme cela est requis pour distribuer la totalité de la solution contenue dans le flacon. De plus, la plupart des silicones sont agréés pour des applications dans le domaine pharmaceutique et alimentaire. Ils ont également l'avantage d'avoir une tension de surface très faible et d'être hydrophobes, antistatiques et anti-salissures ce qui améliore leur maniabilité lors de la fabrication du flacon.

Selon différents modes de réalisation de l'invention, qui pourront être pris ensemble ou séparément :
- l'indice de réfraction dudit tube est d'environ 1,40,
- l'indice de réfraction dudit silicone est compris entre 1,36 et 1,44, de préférence il est d'environ 1,40,
- la différence entre l'indice de réfraction dudit silicone et l'indice de réfraction de ladite solution est inférieure ou égale à 0.04,
- ledit silicone est du polydiméthylsiloxane (PDMS),
- l'indice de réfraction dudit polydiméthylsiloxane est compris entre 1,36 et 1,44, de préférence il est d'environ 1,40,
- la différence entre l'indice de réfraction dudit polydiméthylsiloxane et l'indice de réfraction de ladite solution est inférieure ou égale à 0.04,
- ledit polydiméthylsiloxane est du polydiméthylsiloxane pur ou un alliage de polydiméthylsiloxane,
- ladite solution est une solution alcoolique,
- ladite solution est une solution alcoolique de parfum,
- la transmittance dudit tube est supérieure à 90%, de préférence elle est comprise entre 90 et 99,5%,
- la transmittance dudit silicone est supérieure à 90%, de préférence elle est comprise entre 90 et 99,5%,
- la transmittance dudit polydiméthylsiloxane est supérieure à 90%, de préférence elle est comprise entre 90 et 99,5%,
- ledit tube plongeur comprend une âme, notamment en polyoléfine,
- ledit silicone et/ou polydiméthylsiloxane est sous forme d'un revêtement à la surface de ladite âme,
- ledit tube comprend en outre des nano et/ou microparticules solides,
- ledit tube comprend un revêtement prévu sur ladite âme, ledit revêtement comprenant tout ou partie desdites nano et/ou microparticules solides,
- lesdites nano et/ou microparticules solides ont un indice de réfraction choisi entre 1,3 et 1,5,
- lesdites nano et/ou microparticules solides sont de la silice et/ou du verre,
- ledit tube comprend en outre au moins un polymère semi-cristallin ou amorphe,
- le ou lesdits polymères semi-cristallins ou amorphes sont choisis parmi le polyméthylpentène (TPX), un copolymère cyclo-oléfine (COC), le polyméthacrylate de méthyle (PMMA) ou un mélange,
- la surface dudit tube comprend un marquage,
- ledit marquage est un décor, un texte et/ou une forme géométrique,
- ledit organe de distribution est une pompe,
- ledit tube plongeur est monté sur ledit corps d'organe de distribution par l'intermédiaire d'une partie d'association,
- ledit tube plongeur est issu de matière dudit corps d'organe de distribution,
- ledit flacon comprend d'autres éléments réalisés en un matériau comprenant un silicone de manière à ce que l'indice de réfraction dudit ou desdits éléments soit compris entre 1,36 et 1,44.

L'invention concerne aussi un tube plongeur pour flacon tel que défini précédemment.

L'invention concerne encore un procédé de fabrication d'un tel tube, ledit procédé comprenant une étape d'obtention dudit tube à partir au moins de de silicone, notamment de polydiméthylsiloxane.

Selon différentes caractéristiques de l'invention qui pourront être prises ensemble ou séparément :
- ledit procédé comprend une étape de formation d'une âme du tube et une étape d'application d'un revêtement à la surface de l'âme,
- ledit revêtement comprend le silicone et/ou le polydiméthylsiloxane,
- ladite application dudit revêtement de silicone et/ou polydiméthylsiloxanese est réalisé par pulvérisation, trempage ou dépôt sous vide,
- le polydiméthylsiloxane est du polydiméthylsiloxane solide ou liquide,
- le polydiméthylsiloxane solide est vulcanisable à chaud ou vulcanisable à froid,
- ledit revêtement comprenant des nano et/ou microparticules solides,
- ladite application dudit revêtement comprenant des nano et/ou microparticules solides est réalisé par pulvérisation.
- lesdites nano et/ou microparticules solides ont un indice de réfraction choisi entre 1,3 et 1,5,
- lesdites nano et/ou microparticules solides sont de la silice et/ou du verre,
- ledit tube en particulier ladite âme est obtenu par extrusion et/ou par moulage sous pression,
- ledit procédé comprend une étape de marquage de la surface dudit tube plongeur,
- ledit marquage est appliqué par laser, transfert à chaud, sérigraphie et/ou tampographie,
- ledit marquage est un décor, un texte et/ou une forme géométrique,
- ledit procédé comprend une étape de traitement réduisant le coefficient de frottement dudit tube,
- ledit traitement réduisant le coefficient de frottement est un traitement plasma de surface, une trempe, en particulier en sortie d'extrusion, ou une lubrification de la surface,
- ladite lubrification est faite avec une huile silicone,
- ladite trempe est réalisée en assurant un gradient de température compris entre 200 et 300°C, notamment avec un liquide à température de -40°C à -60°C, en sortie d'extrusion.

D'autres caractéristiques, buts et avantages de l'invention ressortiront de la description qui suit, qui est purement illustrative et non limitative, et qui doit être lue en regard des dessins annexés, sur lesquels :
- les figures 1 et 2 sont des vues de côté d'un flacon selon l'invention, respectivement sans (figure 1) et avec (figure 2) une frette montée sur une bague du flacon ;
- les figures 3 à 8 sont des vues partielles et en coupe longitudinale montrant le montage d'une pompe sur un flacon selon des modes de réalisation de l'invention.

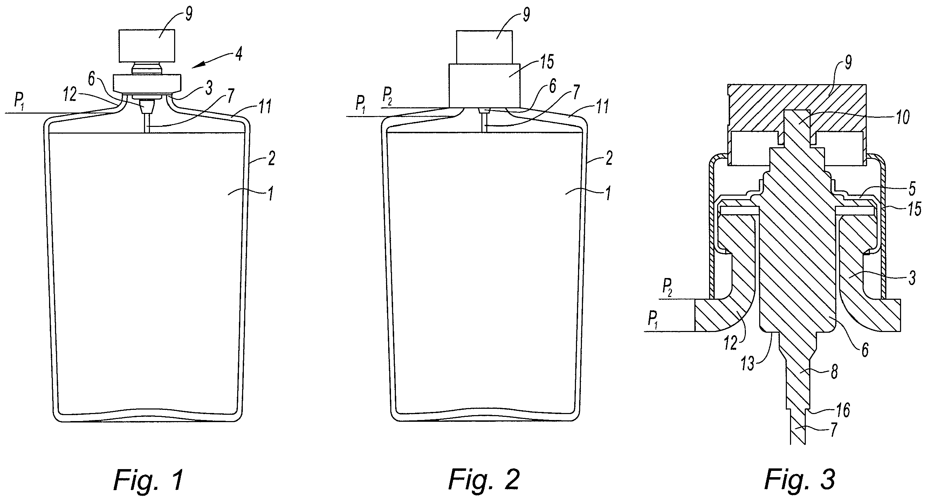

Dans la description, les termes de positionnement dans l'espace sont pris en référence à la position du flacon représentée sur la figure 1.

En relation avec les figures, on décrit ci-dessous un flacon contenant une solution 1, notamment une solution qui est incolore ou faiblement colorée. De préférence il s'agit d'une solution alcoolique, notamment une solution alcoolique de parfum.

Le flacon comprend un corps 2 qui est transparent ou au moins translucide, réalisé, par exemple, en verre ou en matière plastique incolore ou faiblement colorée. Le corps 2 est prévu pour contenir la solution en permettant sa visualisation par l'utilisateur.

Le corps 2 est surmonté par une bague 3 qui est pourvue d'une ouverture sur laquelle un organe de distribution de la solution est associé. Les figures représentent une association de l'organe de distribution 4 sur l'extérieur de la bague 3 au moyen d'une coupelle 5, sans que l'invention ne soit limitée à cette réalisation particulière.

L'organe de distribution 4 comprend un corps 6 d'organe de distribution et un tube plongeur 7 communiquant l'un avec l'autre.

Dans un premier mode de réalisation illustré, l'organe de distribution est une pompe 4. Ledit tube plongeur 7 est monté sur le corps 6 d'organe de distribution (appelé dans ce mode de réalisation corps de pompe) en partie inférieure par l'intermédiaire d'une partie d'association 8. Le tube plongeur 7 est immergé dans la solution 1 pour permettre l'approvisionnement de ladite pompe en solution à distribuer.

Dans un autre mode de réalisation non représenté, ledit tube plongeur peut être issu de matière dudit corps d'organe de distribution. Autrement dit, ledit tube plongeur et le corps d'organe de distribution sont faits d'une seule pièce.

Le corps 6 de pompe et la partie d'association 8 incorporent des organes fonctionnels (non représentés) qui permettent, par actionnement d'un bouton poussoir 9, de distribuer la solution par l'intermédiaire d'un gicleur 10 et d'une buse (non représentée). On connaît un grand nombre d'agencements et de cinématiques de pompes qui permettent la distribution notamment d'un parfum, sur lesquels l'invention peut être mise en oeuvre.

Comme représenté sur les figures, le tube plongeur 7 selon l'invention est sensiblement invisible dans sa partie immergée dans la solution.

Pour ce faire, le tube plongeur 7 comprend un silicone de manière à ce que la différence entre l'indice de réfraction dudit tube 7 et l'indice de réfraction de ladite solution 1 soit inférieure ou égale à 0.04. Ainsi, ledit tube comprend un silicone de manière à ce que l'indice de réfraction dudit tube 7 soit compris entre 1,36 et 1,44, de préférence d'environ 1,40 lorsque l'indice de réfraction de la solution 1 contenue dans le flacon est compris entre 1,37 et 1,39. Autrement dit, l'indice de réfraction dudit tube 7 doit être proche de l'indice de réfraction de la solution 1 contenue dans le flacon.

La transmittance (ou transparence) dudit tube 7 est ainsi supérieure à 90%, de préférence elle est comprise entre 90 et 99,5%.

On choisira par conséquent un silicone dont l'indice de réfraction est compris entre 1,36 et 1,44, de préférence d'environ 1,40. De manière avantageuse, le silicone est du polydiméthylsiloxane (PDMS) dont l'indice de réfraction est d'environ 1,40. Le polydiméthylsiloxane a également l'avantage d'être agréé pharmacopée et alimentarité. Le polydiméthylsiloxane a également l'avantage d'avoir une tension de surface très faible et d'être hydrophobe, anti-statique et anti-salissure. Le polydiméthylsiloxane est également caoutchoutique et présente par conséquent une très grande mémoire de forme et un comportement mécanique hyperélastique Ces caractéristiques particulières permettent une meilleure maniabilité dudit tube 7 lors de la fabrication du flacon, notamment lors de son insertion dans la partie d'association et facilite également sa fabrication et son stockage.

Ledit polydiméthylsiloxane peut être du polydiméthylsiloxane pur ou un alliage de polydiméthylsiloxane. Il peut s'agir de polydiméthylsiloxane solide ou liquide. Dans le cas de polydiméthylsiloxane solide, il est avantageusement vulcanisable à chaud ou vulcanisable à froid.

De manière avantageuse, ledit tube 7 est obtenu par extrusion et/ou par moulage sous pression.

Dans un mode de réalisation, ledit tube comprend en outre des nano et/ou microparticules solides. Lesdites nano et/ou microparticules solides ont de préférence un indice de réfraction choisi entre 1,3 et 1,5 pour ne pas modifier l'indice de réfraction du tube 7 et que celui-ci reste sensiblement invisible dans sa partie immergée dans la solution. Lesdites nano et/ou microparticules solides sont notamment de la silice et/ou du verre.

Ledit tube peut également comprendre un revêtement comprenant des nano et/ou microparticules solides. Il s'agit des nano et/ou microparticules solides décrites précédemment. Dans ce cas, le procédé de fabrication du tube plongeur 7 comprend une étape d'application d'un revêtement comprenant les nano et/ou microparticules solides. Ladite application est réalisée, par exemple, par pulvérisation.

L'utilisation de nano et/ou microparticules solides permet d'améliorer le comportement mécanique du tube plongeur 7, notamment de le rendre plus rigide et ainsi faciliter son insertion dans la partie d'association 8.

De même, pour améliorer le comportement mécanique du tube plongeur 7, ledit tube peut comprendre en outre au moins un polymère semi-cristallin ou amorphe. Celui-ci peut être choisi parmi le polyméthylpentène (TPX), un copolymère cyclo-oléfine (COC), le polyméthacrylate de méthyle (PMMA) ou un mélange.

Une autre solution pour améliorer le comportement mécanique du tube plongeur 7 est de réaliser une étape supplémentaire de trempe en sortie d'extrusion. La dite trempe est réalisée en assurant un gradient de température compris entre 200 et 300°C avec un liquide à température de -40°C à -60°C.

Dans un mode de réalisation particulier, ledit tube plongeur 7 comprend une âme en polyoléfine et ledit silicone et/ou polydiméthylsiloxane est sous forme d'un revêtement à la surface de ladite âme. Le procédé de fabrication du tube plongeur 7 comprend alors une étape d'application d'un revêtement de silicone et/ou polydiméthylsiloxane sur la surface de ladite âme. De préférence, l'application dudit revêtement de silicone et/ou polydiméthylsiloxanese est réalisée par pulvérisation, trempage ou dépôt sous vide de polydiméthylsiloxane. L'utilisation d'un tube en polyoléfine permet d'améliorer le comportement mécanique du tube plongeur 7 tout en conservant les propriétés du polydiméthylsiloxane, c'est-à-dire d'être sensiblement invisible lorsqu'il est immergé dans la solution 1 contenue dans le flacon.

Dans une étape supplémentaire du procédé de fabrication, ledit tube 7 peut également subir un traitement réduisant le coefficient de frottement. Un tel traitement permet, en particulier, de diminuer le coefficient de frottement et faciliter l'insertion du tube 7 dans la partie d'association 8. Le traitement peut être un traitement plasma de surface, une trempe en sortie d'extrusion ou une lubrification de la surface. La lubrification est faite, par exemple, avec une huile silicone.

En variante non représentée, le tube plongeur 7 peut comprendre un marquage. Dans ce cas, le procédé de fabrication peut comprendre une étape de marquage. Il peut s'agir, par exemple de gravure laser, d'impression sur une zone de la périphérie dudit tube, de transfert à chaud, de sérigraphie et/ou de tampographie. Dans des exemples de réalisation, le marquage peut être sous la forme d'un décor, d'un texte, d'une forme géométrique. La combinaison entre le caractère invisible du tube plongeur 7 et la présence du marquage donne alors l'impression visuelle que ledit marquage est en lévitation dans la solution 1.

Pour améliorer l'invisibilité globale de la pompe 4, l'invention prévoit également que le corps 6 de pompe puisse être réalisé dans un matériau transparent ou au moins translucide. De même, lorsque la pompe 4 comprend des organes fonctionnels qui sont potentiellement visibles dans le corps 2 du flacon, en particulier lorsque lesdits organes sont disposés à proximité ou dans la partie d'association 8, l'invention prévoit de réaliser lesdits organes en matériau transparent ou au moins translucide.

Selon une réalisation, la pompe 4 comporte un clapet d'admission de la solution qui est prévu dans la partie d'association 8, ledit clapet comprenant une bille réalisée en matériau transparent ou au moins translucide. Dans le cas où la pompe 4 comprend un ressort en partie inférieure, il est également envisagé de réaliser ce ressort en matériau transparent ou au moins translucide. Par ailleurs, le ressort peut être positionné dans une zone cachée entièrement incluse à l'intérieur de la bague 3 du flacon.

L'invention prévoit en outre que les différents éléments du flacon, notamment ceux réalisés en un matériau transparent ou au moins translucide décrits ci-dessus, soient réalisés dans un matériau comprenant un silicone de manière à ce que l'indice de réfraction dudit ou desdits éléments soit compris entre 1,36 et 1,44, c'est-à-dire le matériau décrit précédemment et utilisé pour fabriquer le tube plongeur 7, en en tous les cas un matériau de même type.

Le flacon comprend une paroi supérieure 11 sur laquelle est formée la bague 3 par l'intermédiaire d'un congé de raccordement 12 dont l'extrémité inférieure est contenue dans un plan sensiblement transversal P1. Ce plan P1 est notamment défini par l'intersection entre la génératrice de la paroi supérieure 11 et la génératrice de l'ouverture de la bague 3.

Pour limiter la visibilité du corps 6 de pompe dans le corps 2 du flacon, l'extrémité inférieure 13 du corps 6 de pompe est située sensiblement dans ou au-dessus du plan transversal P1. Par extrémité inférieure 13 du corps 6 de pompe, on entend la zone qui est adjacente à la partie d'association 8.

Selon les figures 3 à 5 et 8, le corps 6 présente une partie cylindrique supérieure et une partie d'association 8 présentant deux diamètres successifs. Dans ces réalisations, l'extrémité inférieure 13 est la zone comprenant le rebord de raccordement avec la partie d'association 8.

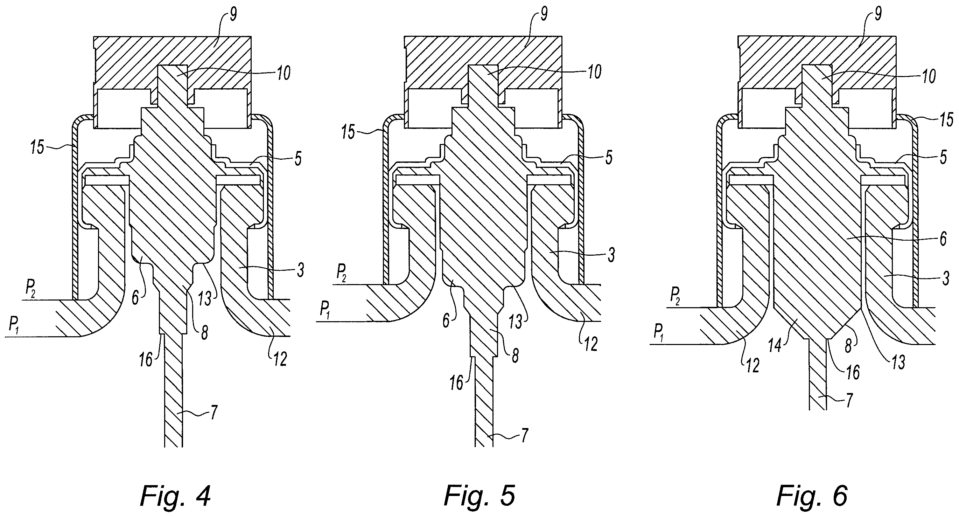

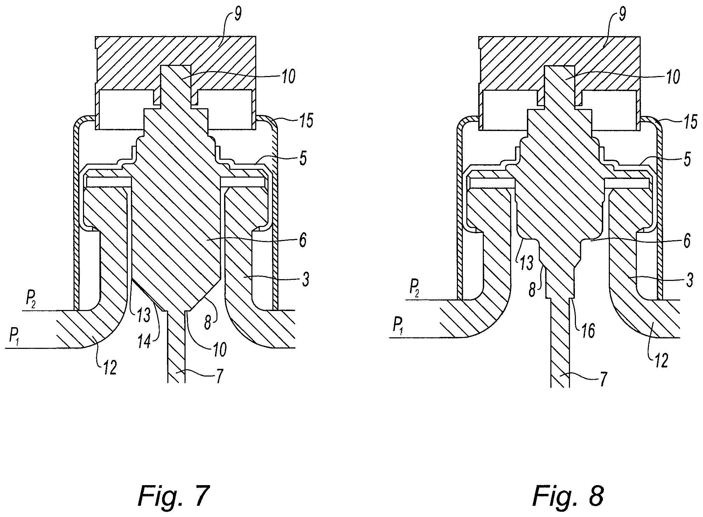

Selon les figures 6 et 7, le corps 6 présente une partie cylindrique supérieure et la partie d'association 8 présente un profil extérieur biseauté 14 qui s'étend à partir de l'extrémité inférieure 13 dudit corps. Dans ces réalisations, la partie d'association 8 peut présenter une dimension réduite et la présence du profil biseauté 14 permet de conférer un effet d'optique qui limite la visibilité de l'extrémité inférieure de la pompe 4.

Selon la figure 2, le flacon comprend en outre une frette 15 qui est montée autour de la bague 3, l'extrémité inférieure de ladite frette étant en appui, dans un plan sensiblement transversal P2, sur la face supérieure de la paroi 11. La frette 15 permet ainsi de masquer la zone d'association de la pompe 4 sur la bague 3.

Selon la figure 3, l'extrémité inférieure 13 du corps 6 est située sensiblement dans le plan P1. Selon les figures 4 et 6, l'extrémité inférieure 16 de la partie d'association 8, c'est-à-dire la zone à partir de laquelle le tube plongeur 7 est visible, est située sensiblement dans le plan P1.

Selon la figure 5, l'extrémité inférieure 13 du corps 6 est située sensiblement dans le plan P2. Selon les figures 7 et 8, l'extrémité inférieure 16 de la partie d'association 8 est située sensiblement dans le plan P2.

## Revendications

1. Flacon contenant une solution, ledit flacon comprenant un corps transparent ou au moins translucide qui est surmonté par une bague (3) pourvue d'une ouverture sur laquelle un organe de distribution (4) de la solution est associé, ledit organe de distribution comprenant un corps (6) d'organe de distribution et un tube plongeur (7) communiquant l'un avec l'autre, ledit tube étant immergé dans la solution (1) pour permettre l'approvisionnement dudit organe de distribution en solution à distribuer, ledit tube plongeur comprenant un silicone de manière à ce que la différence entre l'indice de réfraction dudit tube et l'indice de réfraction de ladite solution soit inférieure ou égale à 0.04.

2. Flacon configuré pour contenir une solution, ledit flacon comprenant un corps transparent ou au moins translucide qui est surmonté par une bague (3) pourvue d'une ouverture sur laquelle un organe de distribution (4) de la solution est associé, ledit organe de distribution comprenant un corps (6) d'organe de distribution et un tube plongeur (7) communiquant l'un avec l'autre, ledit tube étant configuré pour être immergé dans la solution (1) pour permettre l'approvisionnement dudit organe de distribution en solution à distribuer, ledit tube plongeur comprenant un silicone de manière à ce que l'indice de réfraction dudit tube soit compris entre 1,36 et 1,44.

3. Flacon selon l'une quelconque des revendications précédentes, dans lequel ledit silicone est du polydiméthylsiloxane (PDMS).

4. Flacon selon l'une quelconque des revendications précédentes, dans lequel ledit tube plongeur comprend une âme en polyoléfine et ledit silicone est sous forme d'un revêtement à la surface de ladite âme.

5. Flacon selon l'une quelconque des revendications précédentes, dans lequel ledit tube comprend en outre des nano et/ou microparticules solides.

6. Flacon selon la revendication précédente, dans lequel lesdites nano et/ou microparticules solides ont un indice de réfraction choisi entre 1,3 et 1,5.

7. Flacon selon l'une quelconque des revendications précédentes, dans lequel ledit tube comprend en outre au moins un polymère semi-cristallin ou amorphe.

8. Flacon selon la revendication précédente, dans lequel le ou lesdits polymères semi-cristallins ou amorphes sont choisi parmi le polyméthylpentène (TPX), un copolymère cyclo-oléfine (COC), le polyméthacrylate de méthyle (PMMA) ou un mélange.

9. Flacon selon l'une quelconque des revendications précédentes dans lequel ladite solution est une solution alcoolique de parfum.

10. Flacon selon l'une quelconque des revendications précédentes dans lequel ledit organe de distribution (4) est une pompe.

11. Flacon selon l'une quelconque des revendications précédentes dans lequel ledit tube plongeur (7) est monté sur ledit corps (6) d'organe de distribution par l'intermédiaire d'une partie d'association (8).

12. Flacon selon l'une quelconque des revendications 1 à 10 dans lequel ledit tube plongeur (7) est issu de matière dudit corps (6) d'organe de distribution.

13. Flacon selon l'une quelconque des revendications précédentes comprenant d'autres éléments réalisés en un matériau comprenant un silicone de manière à ce que l'indice de réfraction dudit ou desdits éléments soit compris entre 1,36 et 1,44.

14. Procédé de fabrication du tube plongeur pour flacon selon l'une quelconque des revendications 2 à 13 lorsqu'elles ne dépendent pas de la revendication 1, ledit procédé comprenant une étape d'obtention dudit tube à partir au moins de silicone.

15. Procédé de fabrication selon la revendication précédente, dans lequel ledit tube est obtenu à partir au moins de polydiméthylsiloxane solide ou liquide.

16. Procédé de fabrication selon la revendication précédente, dans lequel le polydiméthylsiloxane solide est vulcanisable à chaud ou vulcanisable à froid.

17. Procédé de fabrication selon l'une quelconque des revendications 14 à 16, comprenant une étape de traitement réduisant le coefficient de frottement dudit tube.

18. Procédé de fabrication selon l'une quelconque des revendications 14 à 17, dans lequel ledit tube est obtenu par extrusion et/ou par moulage sous pression.

19. Procédé de fabrication selon la revendication 18, comprenant en outre une étape de trempe dudit tube extrudé, ladite étape de trempe étant réalisée en assurant un gradient de température compris entre 200 et 300°C avec un liquide à température de -40°C à -60°C, en sortie d'extrusion.

## Patentansprüche

1. Flacon, der eine Lösung enthält, wobei der Flacon einen durchsichtigen oder mindestens lichtdurchlässigen Körper umfasst, der von einem mit einer Öffnung versehenen Ring (3) überlagert wird, auf dem ein Verteilungsorgan (4) der Lösung zugeordnet ist, wobei das Verteilungsorgan einen Verteilungsorgankörper (6) und ein Tauchrohr (7), die miteinander kommunizieren, umfasst, wobei das Rohr in die Lösung (1) eingetaucht ist, um die Versorgung des Verteilungsorgans mit zu verteilender Lösung zu ermöglichen, wobei das Tauchrohr ein Silikon umfasst, sodass die Differenz zwischen dem Brechungsindex des Rohrs und dem Brechungsindex der Lösung kleiner als oder gleich 0,04 ist.

2. Flacon, der konfiguriert ist, um eine Lösung zu enthalten, wobei der Flacon einen durchsichtigen oder mindestens lichtdurchlässigen Körper umfasst, der von einem mit einer Öffnung versehenen Ring (3) überlagert wird, auf dem ein Verteilungsorgan (4) der Lösung zugeordnet ist, wobei das Verteilungsorgan einen Verteilungsorgankörper (6) und ein Tauchrohr (7), die miteinander kommunizieren, umfasst, wobei das Rohr konfiguriert ist, um in die Lösung (1) getaucht zu werden, um die Versorgung des Verteilungsorgans mit zu verteilender Lösung zu ermöglichen, wobei das Tauchrohr ein Silikon umfasst, sodass der Brechungsindex des Rohrs zwischen 1,36 und 1,44 umfasst ist.

3. Flacon nach einem der vorstehenden Ansprüche, wobei das Silikon Polydimethylsiloxan (PDMS) ist.

4. Flacon nach einem der vorstehenden Ansprüche, wobei das Tauchrohr einen Steg aus Polyolefin umfasst und das Silikon in Form einer Beschichtung an der Oberfläche des Stegs ist.

5. Flacon nach einem der vorstehenden Ansprüche, wobei das Rohr weiter feste Nano- und/oder Mikropartikel umfasst.

6. Flacon nach dem vorstehenden Anspruch, wobei die festen Nano- und/oder Mikropartikel einen Brechungsindex ausgewählt zwischen 1,3 und 1,5 aufweisen.

7. Flacon nach einem der vorstehenden Ansprüche, wobei das Rohr weiter mindestens ein halbkristallines oder amorphes Polymer umfasst.

8. Flacon nach dem vorstehenden Anspruch, wobei das oder die halbkristallinen oder amorphen Polymere ausgewählt sind aus Polymethylpenten (TPX), einem Cycloolefin-Copolymer (COC), Polymethylmethacrylat (PMMA) oder einem Gemisch.

9. Flacon nach einem der vorstehenden Ansprüche, wobei die Lösung eine alkoholische Parfümlösung ist.

10. Flacon nach einem der vorstehenden Ansprüche, wobei das Verteilungsorgan (4) eine Pumpe ist.

11. Flacon nach einem der vorstehenden Ansprüche, wobei das Tauchrohr (7) auf dem Verteilungsorgankörper (6) mittels eines Zuordnungsstücks (8) befestigt ist.

12. Flacon nach einem der Ansprüche 1 bis 10, wobei das Tauchrohr (7) aus Materie des Verteilungsorgankörpers (6) stammt.

13. Flacon nach einem der vorstehenden Ansprüche, umfassend andere Elemente, die aus einem Material realisiert sind, das ein Silikon umfasst, sodass der Brechungsindex des oder der Elemente zwischen 1,36 und 1,44 umfasst ist.

14. Verfahren zur Herstellung des Tauchrohrs für einen Flacon nach einem der Ansprüche 2 bis 13, wenn sie nicht von Anspruch 1 abhängen, wobei das Verfahren einen Schritt des Erhaltens des Tauchrohrs ausgehend von mindestens Silikon umfasst.

15. Verfahren zur Herstellung nach dem vorstehenden Anspruch, wobei das Rohr ausgehend von mindestens festem oder flüssigem Polydimethylsiloxan erhalten wird.

16. Verfahren zur Herstellung nach dem vorstehenden Anspruch, wobei das feste Polydimethylsiloxan heißvulkanisierbar oder kaltvulkanisierbar ist.

17. Verfahren zur Herstellung nach einem der Ansprüche 14 bis 16, umfassend einen Behandlungsschritt, der den Reibungskoeffizienten des Rohrs verringert.

18. Verfahren zur Herstellung nach einem der Ansprüche 14 bis 17, wobei das Rohr durch Extrusion und/oder durch Druckguss erhalten wird.

19. Verfahren zur Herstellung nach Anspruch 18, weiter umfassend einen Härtungsschritt des extrudierten Rohrs, wobei der Härtungsschritt unter Gewährleistung eines Temperaturgradienten zwischen 200 und 300 °C mit einer Flüssigkeit auf einer Temperatur von -40 °C bis -60 °C, bei Verlassen der Extrusion, realisiert wird.

## Claims

1. Vial containing a solution, said vial comprising a transparent or at least translucent body surmounted by a ring (3) provided with an opening with which a dispensing member (4) of the solution is associated, said member comprising a body (6) of a dispensing member and a dip tube (7) communicating with one another, said tube being immersed in the solution (1) to enable the supply of said dispensing member in solution to be dispensed, said dip tube comprising a silicone so that the difference between the refractive index of said tube and said solution is inferior or equal to 0.04.

2. Vial configured to contain a solution, said vial comprising a transparent or at least translucent body surmounted by a ring (3) provided with an opening with which said dispensing member (4) of the solution is associated, said dispensing member comprising a body (6) of a dispensing member and a dip tube (7) communicating with one another, said tube being configured to be immersed in the solution (1) to enable the supply of said dispensing member in solution to be dispensed, said dip tube comprising a silicone so that the refractive index of said tube ranges from 1.36 to 1.44.

3. Vial according to any one of the preceding claims, wherein said silicone is polydimethylsiloxane (PDMS).

4. Vial according to any one of the preceding claims, wherein said dip tube comprises a polyolefin core and said silicone is in the form of a coating at the surface of said core.

5. Vial according to any one of the preceding claims, wherein said tube further comprises solid nanoparticles and/or microparticles.

6. Vial according to the preceding claim, wherein said solid nanoparticles and/or microparticles have a refractive index selected between 1.3 and 1.5.

7. Vial according to any one of the preceding claims, wherein said tube further comprises at least one semi-crystalline or amorphous polymer.

8. Vial according to the preceding claim, wherein said semi-crystalline or amorphous polymers are selected among polymethylpentene (TPX), a cyclic olefin copolymer (COC), poly(methyl methacrylate) (PMMA) or a mixture thereof.

9. Vial according to any one of the preceding claims wherein said solution is an alcoholic perfume solution.

10. Vial according to any one of the preceding claims wherein said dispensing member (4) is a pump.

11. Vial according to any one of the preceding claims, wherein said dip tube (7) is mounted on said body (6) of the dispensing member by means of a connecting part (8).

12. Vial according to any one of claims 1 to 10 wherein said dip tube (7) is made of the material of said body (6) of the dispensing member.

13. Vial according to any one of the preceding claims comprising other elements made of a material comprising silicone so that the refractive index of said element or elements ranges from 1.36 and 1.44.

14. Method of manufacturing the dip tube for a vial according to any one of the claims 2 to 13, when they are not dependent on claim 1, said method comprising a step whereby said tube is obtained from at least silicone.

15. Manufacturing method according to the preceding claim, wherein said tube is obtained from at least solid or liquid polydimethylsiloxane.

16. Manufacturing method according to the preceding claim, wherein the solid polydimethylsiloxane is hot-cured or cold-cured.

17. Manufacturing method according to any one of claims 14 to 16, comprising a processing step that reduces the friction coefficient of said tube.

18. Manufacturing method according to any one of claims 14 to 17, wherein said tube is obtained by extrusion and/or moulding under pressure.

19. Manufacturing method according to claim 18, further comprising a step whereby said extruded tube is tempered, said tempering step being performed with a temperature gradient ranging from 200°C to 300°C, with a liquid at a temperature ranging from -40°C to -60°C at the extrusion output.
